# EUROPEAN PATENT APPLICATION

(11) **EP 4 512 319 A2**
(43) Date of publication of application: **26.02.2025**
(21) Application number: 24214266.9
(22) Date of filing: 21.04.2020
(51) Int. Cl.: A61B 5/0215

(54) **GUIDEWIRE WITH INTERNAL PRESSURE SENSOR**

(30) Priority: 25.04.2019 US 201962838467 P
(62) Divisional of application: 20794776.3
(71) Applicant: Opsens Inc., Quebec City QC G1P 4S3 (CA)
(72) Inventor: LALANCETTE, Sébastien, Quebec City, G1P 4S3 (CA); BELLEVILLE, Claude, Quebec City, G1P 4S3 (CA); LAFLEUR, Philippe, Quebec City, G1P 4S3 (CA); MERCIER, Jean-Sébastien, Quebec City, G1P 4S3 (CA)
(74) Representative: Boult Wade Tennant LLP

(57) **Abstract**

A pressure guidewire is provided that has a proximal end and a distal end. The pressure guidewire has a proximal section, a sensor housing section, and an intermediate section. The proximal section extends from the proximal end of the pressure guidewire to a distal end of the proximal section. The sensor housing section is disposed adjacent to the distal end of the pressure guidewire. The intermediate section disposed between the proximal section and the sensor housing section. The intermediate section has a proximal end separate from the proximal section. The proximal end can be coupled to the distal end of the proximal section. The pressure guidewire has a tubular body positioned within the intermediate section. A pressure sensor is positioned in the sensor housing section.

## Description

### INCORPORATION BY REFERENCE TO ANY PRIORITY APPLICATIONS

Any and all applications for which a foreign or domestic priority claim is identified in the Application Data Sheet as filed with the present application are hereby incorporated by reference under 37 C.F.R. § 1.57.

### BACKGROUND

### Field

This application is directed to advancements in pressure guidewire technology.

### Description of the Related Art

Guidewires are known for delivering catheters to many vascular locations in the body. Access to remote and tortuous vasculature is facilitated by a combination of mechanical properties such as flexibility, pushability and torqueability.

Coronary catheters can track over simple coronary guidewires to coronary vasculature and can be used to position treatment devices dilatation balloons and stents. Some coronary guidewires are also able to measure blood pressure in the segment of the coronary vasculature. Upon measurement of blood pressure, a treatment diagnosis can be used to guide the treatment to be performed. For example, a measurement such as fractional flow reserve (FFR) can be used to determine which patients should be treated with a balloon, a stent or other approach.

While pressure measuring guidewires have been described and even marketed for many years, such devices can be improved.

### SUMMARY

A need exists for more robust pressure guidewires. Pressure guidewires are very thin and yet contain sophisticated devices in complex assemblies. The assembly process and requirements of use can lead to fracture and other failure modes. Thus, such guidewires should be configured with robust connections between different functional parts. Such guidewires should be made with junctions that preserve delicate structures that measure pressure. Such guidewires should be configured to enhance kink resistance.

In one embodiment, a pressure guidewire is provided that includes a shaft tube assembly, a hypotube, and a tip pressure sensor. The shaft tube assembly can have a proximal section, a middle section, and a sensor housing section. The proximal section can have a first tubular body. The first tubular body can have a proximal end, a distal end, a proximal outside surface and a proximal inside surface. The proximal inside surface can enclose a proximal portion of a central lumen. The proximal outside surface can comprise or form an outer surface of the pressure guidewire. The middle section can have a proximal end, a middle section outside surface, and a middle section inside surface. The middle section inside surface can be disposed about a space within the pressure guidewire. The proximal end of the middle section can be separate from the distal end of the proximal section. The proximal end of the middle section can be coupled to the distal end of the proximal section. The sensor housing section can extend distally relative to the middle section. The hypotube can have a proximal end portion and a distal end portion. The hypotube can extend through the space about which the middle section inside surface is disposed. The proximal end portion of the hypotube can be coupled with the distal end of the proximal section. The distal end portion of the hypotube can be coupled to the sensor housing. The tip pressure sensor can be positioned in the sensor housing section.

In another embodiment, a pressure guidewire is provided that has a proximal end and a distal end. The pressure guidewire has a proximal section, a sensor housing section, and an intermediate section. The proximal section extends from the proximal end of the pressure guidewire to a distal end of the proximal section. The sensor housing section is disposed adjacent to the distal end of the pressure guidewire. The intermediate section disposed between the proximal section and the sensor housing section. The intermediate section has a proximal end separate from the proximal section. The proximal end can be coupled to the distal end of the proximal section. The pressure guidewire has a tubular body and a pressure sensor. The tubular body has a proximal end portion and a distal end portion. The tubular body is positioned within the intermediate section. The pressure sensor is positioned in the sensor housing section. The pressure sensor has a signal conductor disposed proximally of the sensor housing through the tubular body.

The pressure guidewire provides more flexibility in the intermediate section than the proximal section. In one example, a wall thickness of the pressure guidewire is less in the intermediate section than in the proximal section. In one example, the pressure guidewire provides a stepped lumen profile. In one example, the wall thickness of the pressure guidewire is less in the intermediate section than in the proximal section and the pressure guidewire provides a stepped lumen profile.

A thinner wall section can allow a tubular body, e.g., a hypotube, to be disposed in the intermediate portion of the assembly. The tubular body, e.g., the hypotube, can have a smaller outside diameter that provides more flexibility than the larger outside diameter and thicker wall of the proximal section.

In some examples, the sensor that makes pressure measurements includes a micro-electromechanical systems (MEMS) devices which are very small and also very delicate. The assembly of the MEMS device in the pressure guidewires must be carefully done to reduce potential for damage to the MEMS device and/or to sources of measurement error that can arise due to damaging the MEMS structure.

In some cases, the guidewire assembly includes a tip assembly that includes an atraumatic tip, a core wire and a coil structure. The atraumatic tip can be coupled to the core wire by a suitable technique, such as by welding. The core wire can be provided with a heat shield or heat sink to contain heat added to the structure to maintain the heat affected zone away from nearby corewire smaller sections.

In another embodiment a guidewire assembly is provided that includes a proximal section and a distal section. The distal section extends distally of the proximal section. The distal section has an exterior metal body portion, a sensor assembly, and a metal ring member. The sensor assembly has a sensor body and a signal conductor coupled with the sensor body. The sensor assembly is disposed through the exterior body portion. The metal ring member is disposed between the exterior metal body portion and the signal conductor of the sensor assembly. The exterior metal body is joined to the metal ring member providing two metal layers around the sensor assembly.

In another embodiment a method of forming a guidewire assembly is provided. A sensor body is coupled to a metal ring member. The metal ring member is disposed within an exterior metal body. A portion of an exterior surface of the metal ring member and a portion of an interior surface of the exterior metal body are joined.

In another embodiment, a guidewire assembly is provided that includes a proximal section, a distal portion, and a junction. The proximal section has a proximal end and a distal end. The distal portion has a proximal end coupled with the distal end of the proximal section. A detector is disposed at or adjacent to a distal end of the distal portion. The junction includes the distal end of the proximal section and the proximal end of the distal portion. The junction has an enhanced ductility zone. The enhanced ductility zone includes a length of the distal portion including the proximal end thereof, a length of the proximal section including the distal end thereof, or a length of the distal portion including the proximal end thereof and a length of the proximal section including the distal end thereof.

In another embodiment, a method is provided for forming a pressure guidewire. In the method, a proximal body is provided. The proximal body has a first tubular wall that has a first wall thickness and a lumen of a first diameter. A distal body is provided that has a second tubular wall that has a second wall thickness and a lumen of a second diameter. The first diameter is smaller than the second diameter. The first wall thickness is greater than the second wall thickness. A distal end of the proximal body is coupled to a proximal end of the distal body to provide a continuous assembly from proximal of the distal end of the proximal catheter body to distal of the proximal end of the distal catheter body. Heat is applied to the continuous assembly after coupling, e.g., after welding, to enhance the ductility of at least a portion of the continuous assembly disposed at a location from proximal of the distal end of the proximal body to distal of the proximal end of the distal body.

In another embodiment, a pressure guidewire is provided that has a shaft tube assembly, a pressure sensor disposed in a distal portion of the shaft tube assembly, and a tip assembly. The pressure sensor is coupled with a signal conduit to convey pressure signals to a processor. The tip assembly includes a core wire and an atraumatic tip. The core wire has a proximal end coupled to a distal portion of the shaft tube assembly and an elongate tapered body having a lesser diameter toward a distal end thereof. The atraumatic tip portion has a proximal end coupled with a distal end of the core wire and a rounded distal end. The proximal end is configured to restrain heat gain at the distal end of the core wire to prevent a change in material properties in the distal end of the core wire.

In another embodiment, a method of forming a pressure sensing guidewire is provided. A shaft tube assembly is provided that has a distal portion with a pressure sensor disposed therein and a distal end. A proximal end of a core wire is coupled with the distal end of the shaft tube assembly. The wire has an elongate tapered body having a smaller size toward a distal end thereof than adjacent to a proximal end thereof. The core wire has a tip member disposed at the distal end of the elongate tapered body. A coil is positioned over the core wire. The coil is coupled to a proximal portion of the core wire. The tip member is heated to melt a distal portion thereof to form an atraumatic tip portion having a convex shape. The tip member has sufficient heat capacity to prevent material property changes in the core wire while allowing a distal portion to be formed having the convex shape following heating.

### BRIEF DESCRIPTION OF THE DRAWINGS

These and other features, aspects and advantages are described below with reference to the drawings, which are intended for illustrative purposes and should in no way be interpreted as limiting the scope of the embodiments. Furthermore, various features of different disclosed embodiments can be combined to form additional embodiments, which are part of this disclosure. In the drawings, like reference characters denote corresponding features consistently throughout similar embodiments. The following is a brief description of each of the drawings.
FIG. 1 is a schematic diagram showing blood vessels with a cut-out portion in which a pressure guidewire is inserted and, spaced proximally therefrom, a guide catheter located proximally of the cut-out portion, e.g., in an aorta of a patient;
FIG. 2 is a schematic view of a diagnostic system that can include any one of the pressure guidewire embodiments disclosed herein;
FIG. 3 is a side view of an embodiment of a pressure guidewire according to one embodiment disclosed herein;
FIG. 4 is a cross-sectional view in a middle section of the pressure guidewire of FIG. 3 taken at the section plane 4 - 4;
FIG. 5 is a cross-sectional view in a proximal section of the pressure guidewire of FIG. 3 taken at the section plane 5 - 5;
FIG. 6 is a longitudinal cross-section of the pressure guidewire of FIG. 3 taken at the section plane 6-6;
FIG. 7 is a cross-sectional view in a sensor housing section of the pressure guidewire of FIG. 3 taken at the section plane 7 - 7 shown in FIG. 6;
FIG. 8 is a detail view of a portion of the longitudinal cross-section of FIG. 6 showing details of the sensor housing section and a tip assembly;
FIG. 9 shows an exploded view of the tip assembly, showing an atraumatic tip member schematically;
FIG. 10 is a detail view of a portion of the longitudinal cross-section of FIG. 6 showing details of a section disposed between the sensor housing section and a proximal section;
FIG. 11 is an enlarged view of a portion of the view of FIG. 10 showing an outer tubular member over a spiral portion;
FIG. 12 illustrates an approach to coupling the proximal section of the pressure guidewire to the middle section thereof;
FIG. 13 is a view similar to FIG. 3 showing two additional cross-sections related to illustrate additional embodiments;
FIG. 14 is a cross-sectional view of section plane 9-9 in FIG. 13 according to one embodiment;
FIG. 15 is a cross-sectional view of section plane 9-9 in FIG. 13 according to another embodiment;
FIG. 16 is a cross-sectional view of section plane 9-9 in FIG. 13 according to another embodiment;
FIG. 17 is a cross-sectional view of section plane 9-9 in FIG. 13 according to another embodiment;
FIG. 18 is a cross-sectional view of section plane 9-9 in FIG. 13 according to another embodiment;
FIG. 19 is a cross-sectional view of section plane 9-9 in FIG. 13 according to another embodiment;
FIG. 20 is a cross-sectional view of section plane 8-8 in FIG. 13 according to one embodiment;
FIG. 21 is a cross-sectional view of section plane 8-8 in FIG. 13 according to one embodiment; and
FIG. 22 is a cross-sectional view of section plane 8-8 in FIG. 13 according to one embodiment.

### DETAILED DESCRIPTION OF THE PREFERRED EMBODIMENT

This application is directed to improved design and construction techniques for pressure guidewires. Such techniques provide robust connections between separate structures enhancing and fracture resistance. Such techniques provide connections that protect delicate structures from damage caused by stress concentration resulting from material degradation within localized heat affected zone(s), such as can arise during welding and other heat generating manufacturing steps.

### I. OVERVIEW OF PRESSURE WIRE SYSTEMS AND THEIR USE

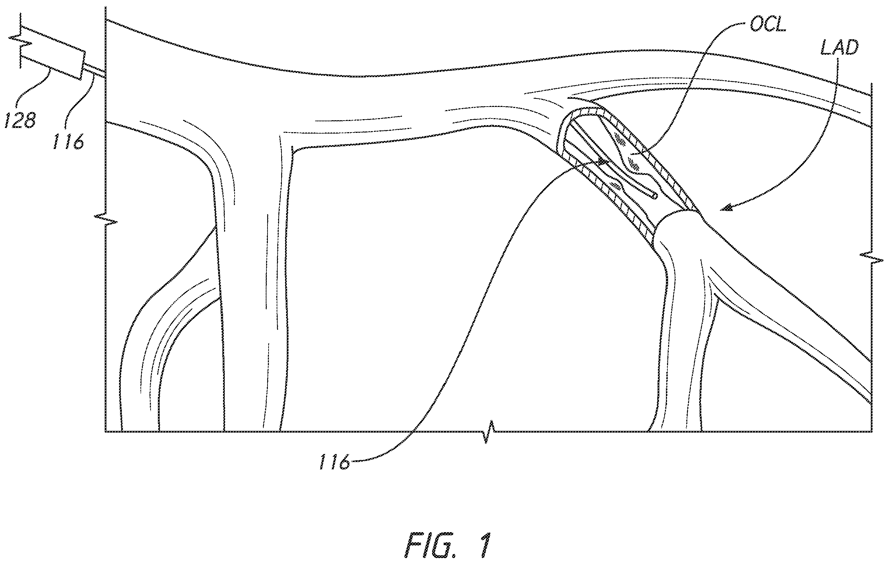

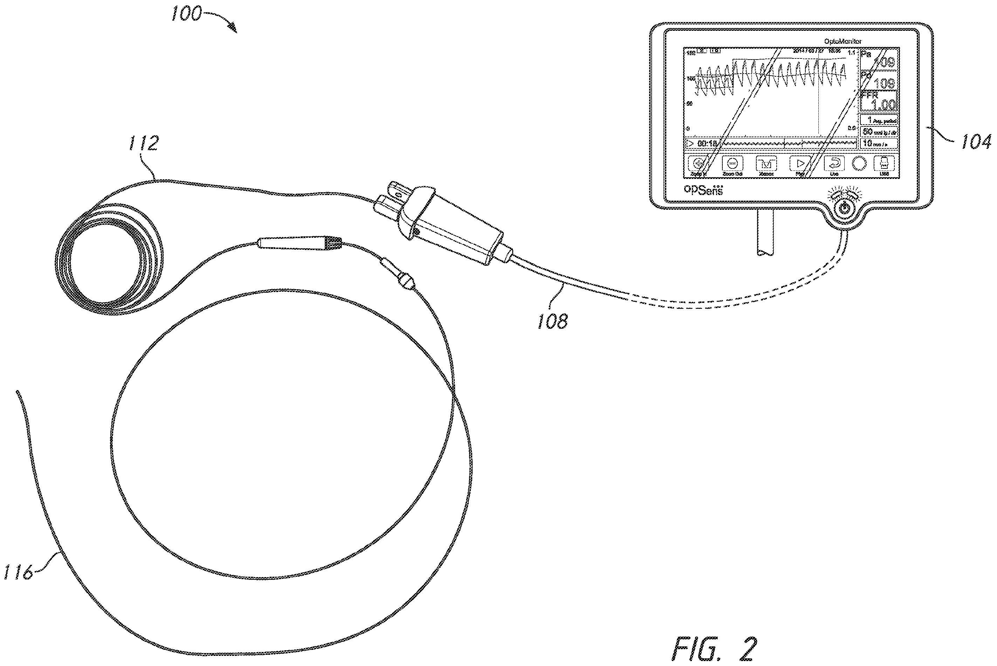

FIGS. 1 and 2 illustrate a lesion diagnostic system 100 and the use thereof in the vasculature of a patient. FIG. 1 illustrates the left side coronary vasculature with a pressure guidewire 116 disposed in a proximal portion of a left anterior descending artery (LAD). The pressure guidewire 116 is positioned in the left anterior descending artery LAD with a distal portion thereof distal to an occlusion OCL. The pressure guidewire 116 is positioned through a guide catheter 114 that can be positioned in the aorta, for example. The blood flow in the left anterior descending artery LAD is on average from proximal to distal, through the occlusion OCL and over the distal tip of the pressure guidewire 116 when the guidewire 116 is placed as shown. The occlusion OCL obstructs flow to at least some extent. The lesion diagnostic system 100 is configured to determine whether the vessel is obstructed to an extent that balloon angioplasty, a stent or other catheter intervention ought to be performed.

FIG. 2 shows that the pressure guidewire 116 disposed at a distal end of the diagnostic system 100 and a monitor assembly 104 is positioned at an end opposite of the pressure guidewire 116 in the system 100. The monitor assembly 104 can be disposed at a proximal end of the diagnostic system 100. The distal end of the diagnostic system 100 is the end that is adapted to be positioned in the patient, e.g., in the left anterior descending artery LAD as discussed above. The proximal end of the diagnostic system 100 includes the portion near the cardiologist and in the case of the monitor assembly 104 outside the patient. One or more devices can be used to connect the pressure guidewire 116 to the monitor assembly 104. As discussed more below, the pressure guidewire 116 can be configured to measure pressure using an optical sensor. In such embodiments the pressure guidewire 116 can be coupled to the monitor assembly 104 by a handle 108 and a fiber optic interface cable 112. The fiber optic interface cable 112 conveys the optical signal from the pressure guidewire 116 to the monitor assembly 104. The handle 108 couples the fiber optic interface cable 112 to the monitor assembly 104.

In one approach, the monitor assembly 104 and the handle 108 are reusable components of the diagnostic system 100. The pressure guidewire 116, the fiber optic interface cable 112 or both can be disposable components. In some variations, the handle 108 and the fiber optic interface cable 112 are a single unit.

### II. EXAMPLE PRESSURE GUIDEWIRES

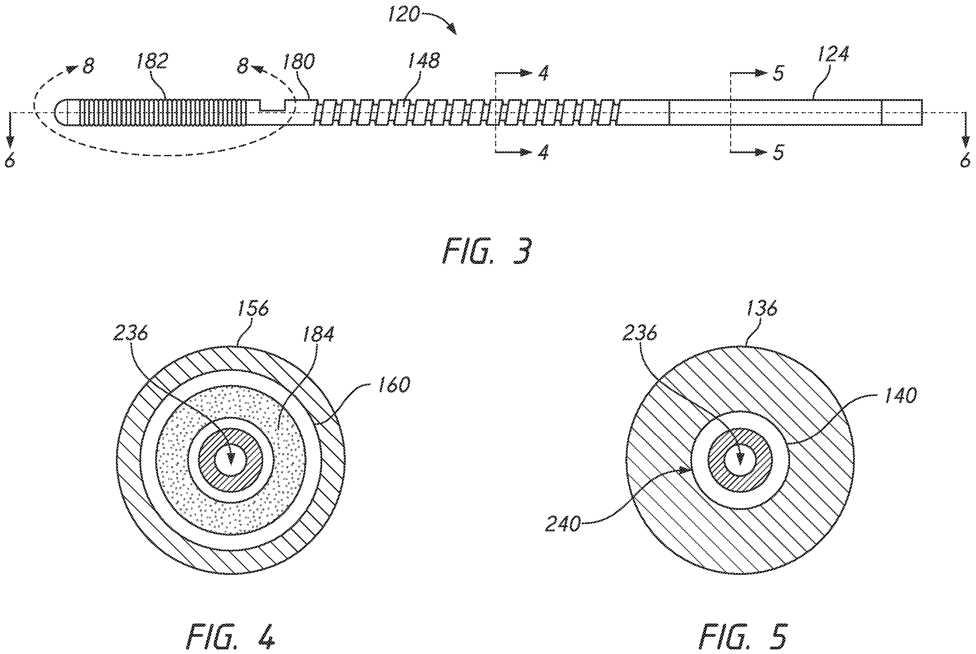

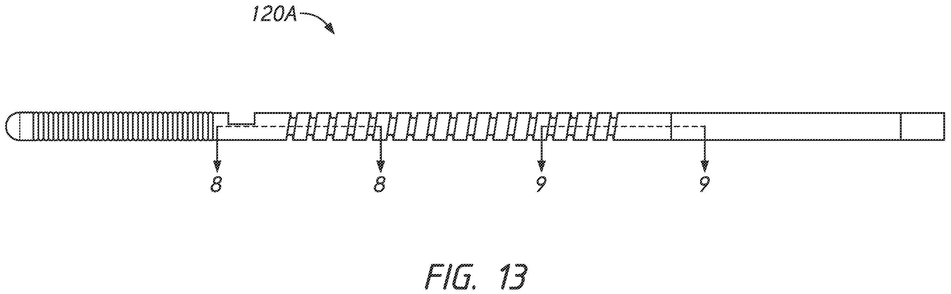

FIG. 3 shows the overall configuration of a pressure guidewire 116 according to one embodiment. The pressure guidewire 116 can include a shaft tube assembly 120 that includes a proximal section 124, a middle section 148, a sensor housing section 180, and a tip assembly 182. Shaft tube assembly 120A, illustrated in FIGS. 13-21 and corresponding text describe additional embodiments of the pressure guidewire 116. These components extend along and define outer surfaces of the pressure guidewire 116. The construction and design of the pressure guidewire 116 is improved by providing two or more components forming the outer surface of the wire, e.g., in the proximal section 124 and in the middle section 148. As discussed below, the pressure guidewire 116 is formed by joining a first annular face 224 of a proximal tubular member to a second annular face 228 of a distal tubular member. An advantageous connection is provided between the proximal section 124 and the middle section 148. An advantageous connection is provided in the sensor housing section 180. Improved assemblies are provided in the tip assembly 182.

FIGS. 3, 5, and 6 show features of the proximal section 124. The proximal section 124 includes a first tubular body 126 that extends between a proximal end 128 and a distal end 132 of the proximal section 124. The tubular body 126 includes a proximal outside surface 136 and a proximal inside surface 140. The proximal outside surface 136 of the tubular body 126 defines a proximal portion of an outside surface of the pressure guidewire 116. The diameter of the proximal outside surface 136 is configured to enable the guidewire 116 to enable a therapy catheter to be slideably advanced thereover, e.g., between the proximal outside surface 136 and an inside surface of the guide catheter 114. The proximal outside surface 136 can be between 0.2mm and 2.0mm, e.g., in one embodiment about 0.36mm.

The proximal inside surface 140 can be sized to enable a signal conductor 220 extend therethrough. The signal conductor 220 can extend through a central lumen 144 disposed within the proximal inside surface 140. The proximal inside surface can have a size close to that of the signal conductor 220. The thickness of the wall of the proximal section 124 between the outside surface 136 and the inside surface 140 can be about 0.1mm. An inner diameter of the proximal section 124 can be between 0.05mm and 0.25mm, e.g., about 0.16mm in one embodiment. The size of the lumen 144 can be between 0.05mm and 0.25mm, e.g., about 0.16mm in one embodiment. In one embodiment, the diameter of the lumen 144 can be less than the combined thickness of the wall of the proximal section 124 on opposite sides of the lumen 144. The diameter of the lumen 144 can be between 20% and 100% of the combined thickness of the wall of the proximal section 124 on opposite sides of the lumen 144. The diameter of the lumen 144 can be between 60% and 90% of the combined thickness of the wall of the proximal section 124 on opposite sides of the lumen central lumen 144, e.g., about 80% in some examples. A clearance gap between the inside surface 140 and an outside surface of the signal conductor 220 can be at least about 0.0127mm, e.g., about 0.025 mm.

The proximal section 124 provides an improved proximal section configuration in enabling the signal conductor 220 to be centrally disposed in a central lumen 144 of the pressure guidewire 116. The proximal section 124 can be configured to provide sufficient support in the proximal section 124 such that the pressure guidewire 116 can be assembled without any core wire or similar reinforcement structures in the proximal section 124. The thickness of the wall of the proximal section 124 provides sufficient mechanical performance, e.g., pushability, torqueability, and kink resistance without additional reinforcement. The proximal section 124 can include a continuously concave surface 240 disposed around signal conductor 220. A continuously concave surface 240 can be formed by the proximal inside surface 140 of the proximal section 124 in one embodiment. The continuously concave surface 240 can be separated from the signal conductor 220 by only an annular gap therebetween.

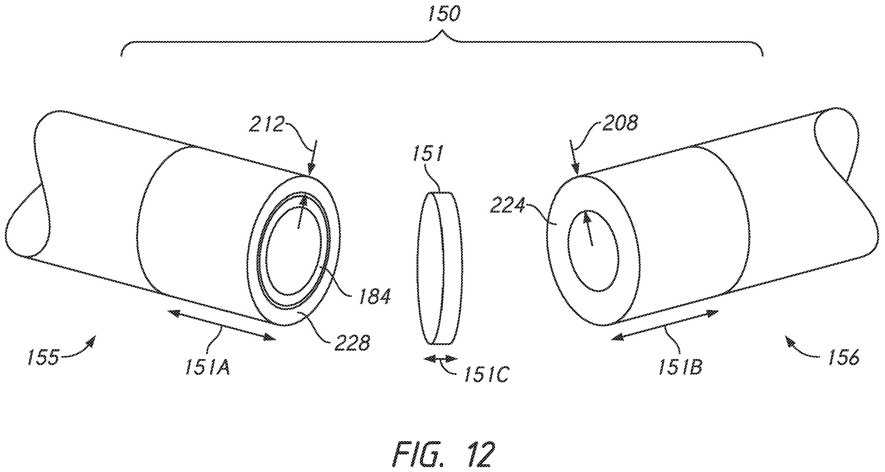

The proximal section 124 can be configured such that the tubular body 126 has a first thickness 208 between the proximal outside surface 136 and the proximal inside surface 140. The first thickness 208 can be sufficient to provide the support needed to avoid any kinking or fracture that would render the pressure guidewire 116 inoperative. The first thickness 208 can be substantially constant from the proximal end 128 to the distal end 132. FIG. 12 shows that the tubular body 126 can have a first annular face 224 at the distal end 132. The first annular face 224 is configured to couple with a second annular face 228 of the middle section 148 at a junction 150 between the proximal section 124 and the middle section 148 as discussed further below.

FIGS. 3, 4, and 6 show details of the middle section 148. The middle section 148 includes a proximal end 152 and a tubular body 149 that extends from the proximal end 152 to a distal end. The distal end can be coupled the sensor housing section 180 in one embodiment. In another embodiment, the distal end of the middle section 148 can extend into and form a portion, e.g., the outer surface, of the sensor housing section 180.

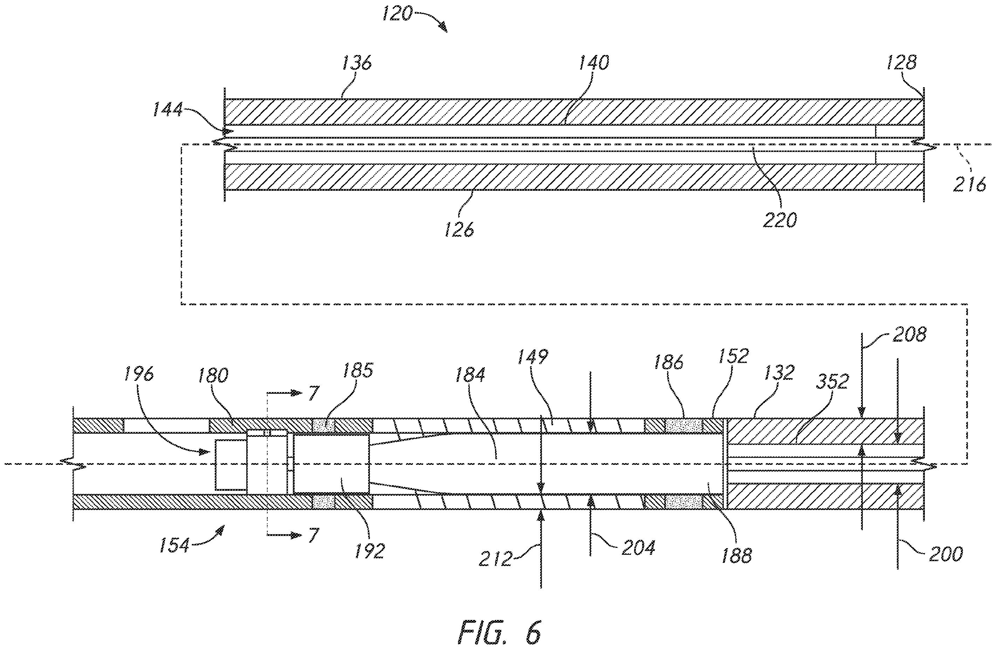

The tubular body 149 has a middle section outside surface 156 and a middle section inside surface 160. The middle section outside surface 156 can form a portion of an outside surface of the pressure guidewire 116. FIGS. 4 and 6 show that the middle section outside surface 156 and the proximal outside surface 136 of the proximal section 124 can form a substantially continuous outer surface of the pressure guidewire 116 from the distal end 132 of the proximal section 124 to the proximal end 152 of the middle section 148. The tubular body 149 can have an outside diameter defined by the middle section outside surface 156 that is the same or substantially the same as the diameter of the proximal outside surface 136. The middle section 148 can have an outside diameter between 0.2mm and 2.0mm, e.g., about 0.36mm, about 0.46 mm, about 0.89mm, and about 0.97mm in various embodiments.

The tubular body 149 can be configured to enable the pressure guidewire 116 to have enhanced flexibility in the middle section 148.

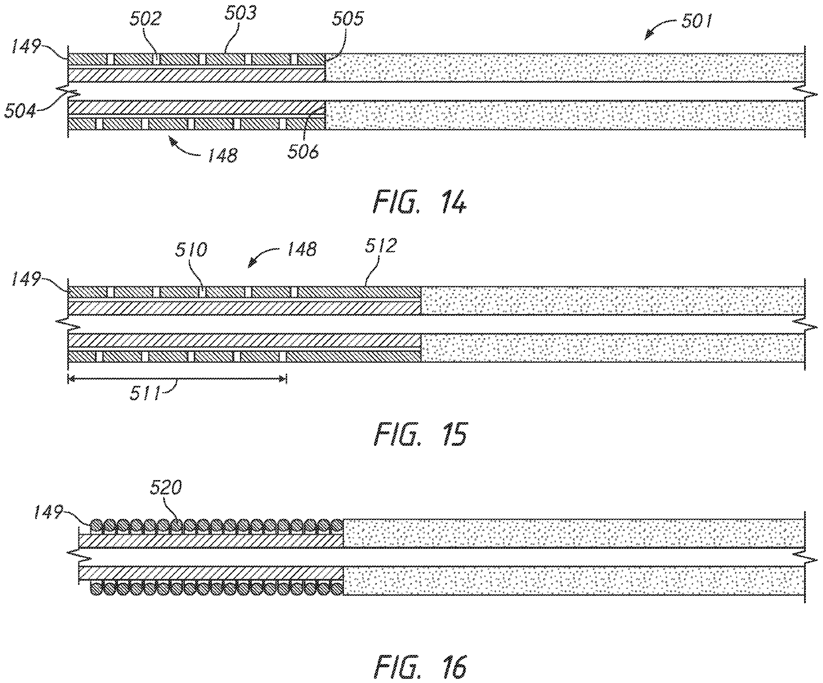

The middle section 148 can be made significantly more flexible by forming at least a portion of the tubular body 149 into a discontinuous configuration, e.g., a ribbon, a spiral, a coil or other suitable configuration. A ribbon configuration (FIG. 14) can be formed by spiraling ribbon 503, preferably a square or rectangular ribbon, with spacing 502. Ribbon can preferably be made of stainless steel, cobalt chrome or other metal, or it can be made of polymer ribbon such as by way of examples Teflon^{™}, polyimide(PI), polyvinyl chloride (PVC), polypropylene (PP), polyethylene (PE), polystyrene (PS), nylon, polyethylene terephthalate (PET), polycarbonate (PC), acrylonitrile butadiene (ABS), polyetheretherketone (PEEK), polyether block amide (PEBA) and polyurethane (PU). The ribbon or other metal structures of the pressure guidewire 116, including the proximal section 124, can include materials such as stainless steel, such as 304V, 304L, and 316LV stainless steel, 17-7PH stainless steel, mild steel, nickel-titanium alloy such as linear-elastic and/or super-elastic nitinol. Also, other nickel alloys such as nickel-chromium-molybdenum alloys (e.g., UNS: N06625 such as INCONEL^{®} 625, UNS: N06022 such as HASTELLOY^{®} UNS: N10276 such as HASTELLOY^{®} C276.RTM., other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N.RTM. and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like), platinum enriched stainless steel, titanium, combinations thereof, and the like, or any other suitable material can be used. Proximal end face 505 of ribbon 503 can be flattened to adapt to distal end face of proximal section 501. A spiral configuration (Fig. 15) can be made by laser cutting a spiral shaped gap 510 in at least a portion 511 of the middle section 148 preferably made of stainless, other metal or polymer tubular body. A coil configuration (Fig. 16) can be made by coiling a wire 520 preferably made of stainless steel, platinum, palladium or other platinum or palladium based metal, other metal or polymer to form the middle section

The middle section 148 can be reinforced to enhance or even optimize torque transfer, pushability, support, kink and/or fracture resistance of the pressure guidewire 116 in the middle section 148. In one embodiment, a hypotube 184 can be positioned in the middle section 148. FIG. 6 shows that the hypotube 184 can extend from proximal of a ribbon, spiral or coil portion of the tubular body 149 to distal of the ribbon, spiral or coil portion. The hypotube 184 can have an inside surface forming a portion of the central lumen 144 of the pressure guidewire 116. The inside surface of the hypotube 184 can form a diameter that is substantially the same as the inside diameter of the tubular body 126 of the proximal end 128. Preferably, the inside diameter of the hypotube 184 can be made to accommodate the outside diameter of the signal conductor 220. The inside diameter of the hypotube 184 can be between 0.05mm and 0.2mm, e.g., about 0.13mm in one embodiment. The central lumen 144 can have a substantially constant inner diameter in one embodiment, e.g., from the proximal end to the distal end of the pressure guidewire 116. In one variation, the inside diameter of the hypotube 184 can be smaller than the inside diameter of the tubular body 126 such that a clearance between an outside surface of the signal conductor 220 and the inside surface of the hypotube 184 is smaller than a clearance between the outside surface of the signal conductor 220 and the inside surface of the tubular body 126. A greater clearance between the signal conductor 220 and tubular body 126 can allow the optical fiber 236 to be more easily advanced through the tubular body 126. On the other hand, the inside diameter of the hypotube 184 can be smaller than the inside diameter of the tubular body 126 such that hypotube 184 wall thickness can be made thicker.

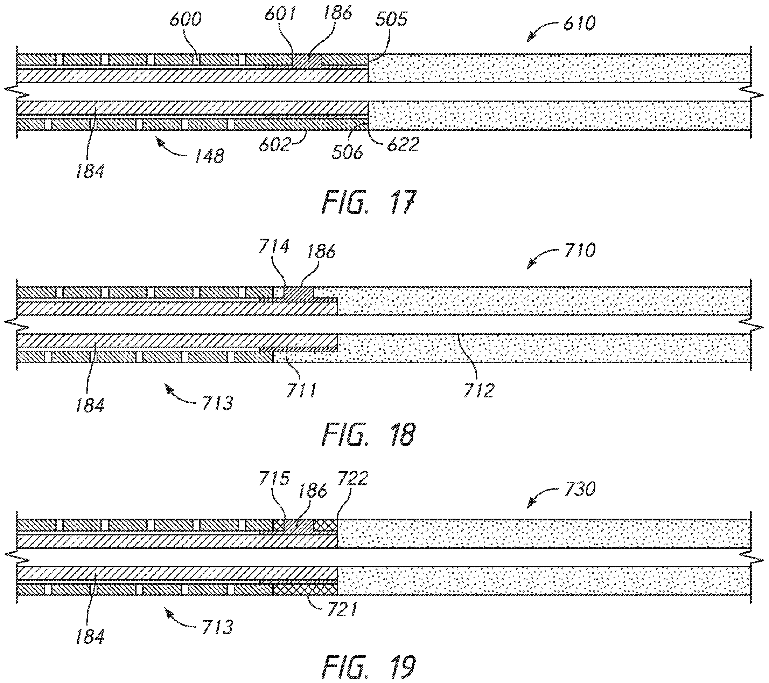

In another embodiment shown in Fig. 17, the middle section 148 is made by cutting a spiral 600 along at least a portion of the middle section. The cut 600 is preferably made throughout the whole thickness of the wall of the tubular body in the middle section 148. In one variation, the cut 600 partially goes through the wall of the tubular body to modify the flexibility of the middle section 148. The proximal end face 505 of middle section 148 can be butt coupled to distal end face of proximal section 610 to form a junction 622. Butt coupling assembly method includes direct laser welding, soldering and other methods. Proximal end of hypotube 184 can be joined to proximal end of middle section tubular body 602 by providing adhesive 186 between the outside surface of the hypotube 184 and the inside surface of middle section tubular body 602. The tubular body 602 may include an opening 601 facilitating the migration of adhesive within the proximal region where hypotube is joined to middle section.

In another embodiment shown in Fig. 18, the inside diameter of the proximal section 710 is not constant. More specifically, the inside diameter of the distal portion of the proximal section 711 can be enlarged to accommodate the hypotube 184, the remaining proximal inside diameter 712 being smaller and accommodating the signal conductor, e.g., the optical fiber 236. The enlarged inside diameter portion can be as short as 1 mm or less, it can be as long as 5 mm or more, the length is preferably around 2 to 3 mm long. The enlarged portion can be drilled using a drill bit, a laser beam or other methods known in the art. The hypotube 184 is preferably joined to and within the distal enlarged portion of proximal section 711 using adhesive 186. The distal portion 711 may include an opening 714 to facilitate the migration of adhesive 186 between the outside surface of hypotube and inside surface of enlarged portion of proximal section. The middle section 713 can be made of a ribbon, a spiral or a coil configuration. The proximal end face of the middle section can be butt coupled to the distal end face of proximal section. The proximal portion of the middle section can also be joined to the proximal portion of the hypotube by using adhesive between their respective inside and outside proximal surfaces. The middle section 713 can also be joined by butt coupling to proximal section 711. The middle section 713 can be bonded to hypotube with an adhesive as described above.

In another embodiment shown in Fig. 19, a coupler 721 is attached to the distal end of the proximal section 730. The coupler 721 has an inside diameter to accommodate the hypotube 184. The coupler 721 can be as short as 1 mm or less, it can be as long as 5 mm or more, the length is preferably around 2 to 3 mm long. The proximal end face of the coupler 721 can be butt coupled distal end face of proximal section 730 to form a junction 722. The proximal end of the hypotube 184 can be joined to and within the coupler 721 using adhesive 186. The coupler 721 may include an opening 715 to facilitate the migration of adhesive 186 between the outside surface of hypotube 184 and inside surface of coupler 721. The middle section 713 can be made of or can include a ribbon, a spiral or a coil configuration. The proximal end face of the middle section 713 can be butt coupled to the distal end face of coupler 721. The proximal portion of the middle section can also be joined to the proximal portion of the hypotube 184 by using adhesive between their respective inside and outside proximal surfaces. The middle section 713 can also be joined by butt coupling to proximal section 730. The middle section 713 can be bonded bonding to hypotube 184 with an adhesive as described above.

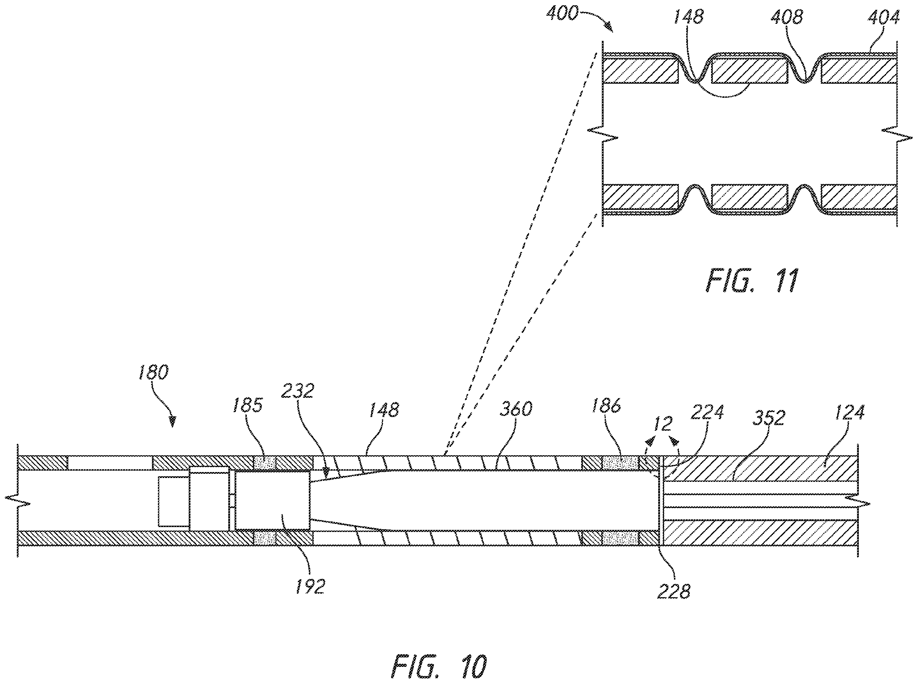

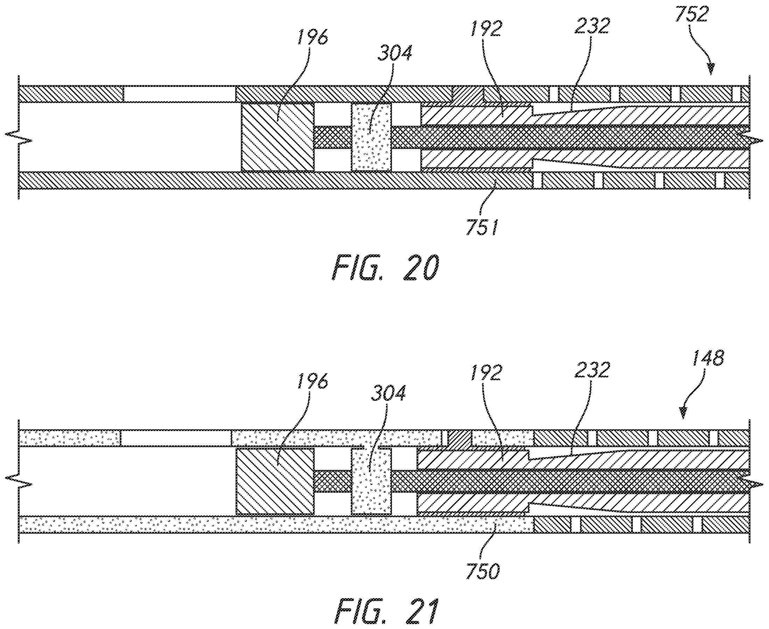

The hypotube 184 can be shaped to provide a varying flexibility along the length of the hypotube 184 and therefore along the length of the middle section 148. Preferably, the outside surface of the hypotube 184 has an increasingly reduced outside diameter forming a tapered portion that is localized toward the distal end of the hypotube. The hypotube 184 can include a distal end portion 192 that is cylindrical and that is enlarged compared to a tapered portion 232 of the hypotube 184 as shown in FIGS 20, 21. The purpose of the enlarged section 192 can be for joining distal end of hypotube 184 to the inside surface of the proximal end of sensor housing 750. The sensor housing 750 can be a separate tubular body from the tubular body of the middle section 148. The enlarged section 192 can also be joined to the inside surface of sensor housing 751 at a proximal end thereof. The sensor housing 750 can be the continuation of tubular body of middle section 752. The hypotube 184 can have a cylindrical portion proximal of a tapered section, as shown in FIGS 6 and 10. The hypotube 184 can be made of a highly elastic or a super elastic material, such as nickel-titanium alloy (nitinol). Other materials that could be used or the hypotube 184 include stainless steel, cobalt-chrome, and other materials with elasticity in the expected strain regime.

The manner of forming the junctions 150, 622, and 722 is important for maintaining the structural integrity of the pressure guidewire 116. The junction 150 can include a junction between the tubular body 126 of the proximal section 124 to the tubular body 149 of middle section 148. The junction 150 can include a junction between the hypotube 184 and the tubular body 149. The junction 150 can include a junction between the hypotube 184 and the tubular body 126 of the proximal section 124. The junction 622 can include a junction between the distal end of proximal section 610 and the proximal end of middle section 602. The junction can include a junction between the distal end of proximal section 710 and the proximal end of tubular body of middle section 713. The junction can include a junction between the distal end of proximal section 730 and the proximal end of coupler 721.The junction can include a junction between the distal end of coupler 721 and the proximal end of tubular body of middle section 713.

In one embodiment an adhesive 185 is provided between an outside surface of the distal end portion 192 of the hypotube 184 and the middle section inside surface 160. A seal, e.g., by way of an adhesive, can be provided between the outside surface of the distal end portion 192 of the hypotube 184 and the middle section inside surface 160. In one embodiment an adhesive 186 is provided between an outside surface of a proximal portion of the hypotube 184 and the middle section inside surface 160, adjacent to the proximal end 152. A seal can be provided between the outside surface of the proximal portion of the hypotube 184 and the middle section inside surface 160, adjacent to the proximal end 152. In one embodiment the adhesive 186 also provides a seal between the outside surface of the proximal portion of the hypotube 184 and the middle section inside surface 160 adjacent to the proximal end 152.

FIG. 12 shows details of a junction 150. The junction 150 can be formed between the proximal section 124 and the middle section 148. The tubular body 126 of the proximal section 124 has a first annular face 224. The tubular body 149 of the middle section 148 has a second annular face 228 or a coupler, such as any of those disclosed herein, e.g., in FIG. 19. The first annular face 224 and the second annular face 228 are secured together at the junction 150. FIG. 12 shows that the first thickness 208 at the first annular face 224 may be greater than the second thickness 212 at the second annular face 228. The first annular face 224 and the second annular face 228 can be joined by any suitable technique. In one embodiment a weld zone 151 is provided between the tubular body 126 of the proximal section 124 and the tubular body 149 of the middle section 148. The weld zone 151 is shown as a short cylinder section mainly for illustration purposes. The weld zone 151 can be a weld line formed when laser welding as the first annular face 224 and the second annular face 228 are held together or adjacent to each other. The junction 150 can comprise a butt junction. The junction 150 can be formed between any two tubular bodies 155 and 156, including between proximal sections 610, 710 and 730 and middle sections 148 and 713 or coupler 721.

In addition to forming the weld zone 151 in connecting two tubular bodies 155 and 156 at the junction 150, in some embodiments the junction 150 is configured to enhance kink or fracture resistance. In some laser welding techniques a laser weld can affect the mechanical properties of welded materials. More specifically, the elastic modulus, tensile strength, yield strength or a combination of the same can be negatively affected within the heat affected zone.. The change in mechanical properties can soften the material. Typical laser welding joint can be very localized, i.e. the heat affected zone can be very localized at the junction. When mechanically challenged, for example if the device is bent within or around the junction, most of the strain (deformation) ends up concentrating in the very localized region of heat affected zone 151C. The risk of fracture therefore increases quite significantly. In one approach a ductility enhancement zone 151A is provided on the tubular body 149 of the middle section 148. The ductility enhancement zone 151A can extend along a length of the tubular body 149 of the middle section 148 from the proximal end 152 toward the distal end of the tubular body 149. The ductility enhancement zone 151A can extend at least about a distance equal to the outer diameter of the middle section 148. The ductility enhancement zone 151A can extend at least about a distance equal to about two times, three times, four times, or five times the outer diameter of the middle section 148. The ductility enhancement zone 151A can extend from the proximal end 152 at least 10% of the distance to the ribbon portion of the middle section 148. The ductility enhancement zone 151A can extend from the proximal end 152 at least 20% of the distance to the ribbon portion of the middle section 148. The ductility enhancement zone 151A can extend from the proximal end 152 at least 30% of the distance to the ribbon portion of the middle section 148. The ductility enhancement zone 151A can extend from the proximal end 152 at least 40% of the distance to the ribbon portion of the middle section 148.

In one embodiment, the junction 150 is configured such that a ductility enhancement zone 151B is provided in the tubular body 126 of the proximal section 124. The ductility enhancement zone 151B is similar to the ductility enhancement zone 151A and can extend from the distal end 132 proximally toward the proximal end 128. The ductility enhancement zone 151B can have a length similar to or the same as the ductility enhancement zone 151A.

In another embodiment, the junction 150 is configured such that a ductility enhancement zone is provided in the coupler 721 and/or in the distal region of proximal section 730. The junction is configured such that a ductility enhancement zone is provided in the region of proximal section 710 where inside diameter suddenly decreases.

In one embodiment, a ductility enhancement zone 151C can be provided at the weld zone 151. In other words, a portion or all of the weld line or zone can be provided with the weld zone 151.

The junction 150 can have a ductility enhancement zone that can include at least a portion of the tubular body 126, at least a portion of the tubular body 149, or at least a portion of the weld zone 151. The weld zone ductility enhancement zone 151 extend from proximal of the first annular face 224 to distal of the second annular face 228. Ductility can be provide above a threshold level from the ductility enhancement zone 151B, through the ductility enhancement zone 151C and into the ductility enhancement zone 151A. The ductility enhancement zone 151C can have a ductility less than an initial (pre-treatment) ductility. The post treatment ductility can be about 90% of the pre-treatment ductility, in some cases between 20 and 90% of the pre-treatment ductility, in some cases between 30 and 80% of the pre-treatment ductility, in some cases between 40 and 70% of the pre-treatment ductility, in some cases between 45 and 60% of the pre-treatment ductility. Ductility can be as measured using a three point bend test, as is known to those skilled in the art.

The hypotube 184 can be secured in the pressure guidewire 116 by one or more adhesive joints as discussed above. The hypotube 184 can be secured in the junction 150 as well. The hypotube 184 can be secured at the weld zone 151. A proximal face of the hypotube 184 can be joined to the first annular face 224 of the tubular body 126. In other words, the second annular face 228 and the proximal face of the hypotube 184 can both be welded to the tubular body 126.

One method for enhancing the ductility of the junction 150 is to provide a localized heat treatment of at least a portion of the pressure guidewire 116 including the junction 150. An example of a heat treatment is to heat the welded region to a temperature above or around the annealing temperature. More specifically, heat treatment can include heating junction 150 to a temperature of or around 1100°C for a short period of time and let it cool in air.

FIGS. 6--8 show details of the sensor housing section 180. The sensor housing section 180 comprises a portion of the pressure guidewire 116 where a sensing device is placed in pressure communication with blood in a blood vessel in the use of the pressure guidewire 116 as discussed in connection with FIGS. 1-2. The sensor housing section 180 includes a tip pressure sensor 196. The tip pressure sensor 196 can include a MEMS sensor unit that is able to detect pressure. The MEMS sensor unit is one example of a detector. The MEMS sensor unit can be a device mounted on a small tubular body made of glass, metal or another material. The MEMS sensor unit can include or can be coupled to the optical fiber assembly. The MEMS sensor unit can be integrated into a sensor body 300. The sensor body 300 can include one or more functions, such as minimizing assembly induced stresses, e.g., by providing a flat bonding surface that allows a thin layer of adhesive retaining a sensor, aligning the sensor within a tube of the pressure guidewire 116, preventing adhesive from reaching the sensor when assembling the sensor to the internal surface of a tubular body, and other functions. The MEMS sensor can employ an optical detection principle. The tip pressure sensor 196 can be disposed in the sensor housing section 180 in a location to be in pressure communication with blood in a vessel. The tip pressure sensor 196 can include a delicate structure that requires a secure connection in the pressure guidewire 116 and also requires the sensor not be damaged in the manufacturing process. The tip pressure sensor 196 can be secured with a ring member 304. The ring member 304 can be secured to the optical fiber 220, nearby and proximal to a sensor body 300 of the tip pressure sensor 196. The ring member 304 can be secured by an adhesive layer 312 disposed between the ring member 304 and the optical figure 220.

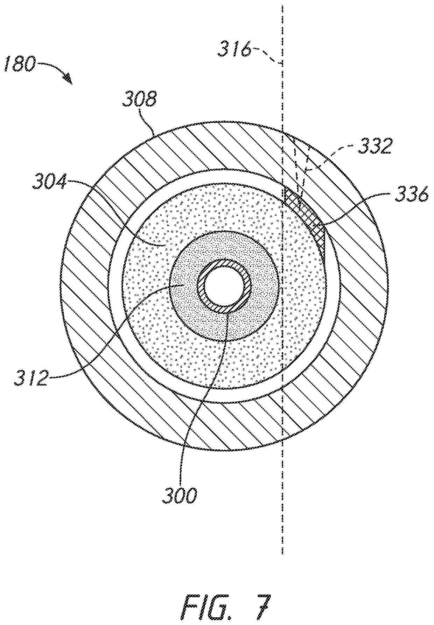

FIG. 7 shows that the adhesive layer 312 can be an annular layer between the ring member 304 and the optical fiber 220. The adhesive layer 312 can be positioned to substantially center the sensor body 300 and the fiber 220 relative to the ring member 304. The ring member 304 can hold the sensor body 300 of the tip pressure sensor 196 securely in position in the sensor housing section 180. FIG. 7 shows that the sensor housing section 180 can have an outer tubular body 308 that can be separate from or can be an integral extension of the tubular body 149 of the middle section 148. The outer tubular body 308 can have the ring member 304 disposed therein. The ring member 304 can be substantially centered in the outer tubular body 308. The ring member 304 can be held in the outer tubular body 308 by a material bridge 336 extending between an outside surface of the ring member 304 and an inside surface of the outer tubular body 308. The material bridge 336 can be a separate material, such as an adhesive, in one embodiment. In another embodiment, the material bridge 336 is a fused weld line between the ring member 304 and the outer tubular body 308.

The ring member 304 can be made of various materials such as polymer, glass or metal. In one embodiment, the ring member 304 can include a metal ring. The metal ring can be bonded to a glass structure such as a glass ring that can be part of the sensor body 300. In one case, the ring member 304 can include a metal ring that is bonded to a glass ring that is further coupled to the sensor body 300 which may or may not include another glass ring for holding a MEMS sensor unit or structure. The ring member 304 can be made of a material that can be fused welded/bonded to the inside surface of the sensor housing by way of localized sensor housing heating. Preferably, the ring member is made of a metal that can be fused or welded to the sensor housing such as stainless steel. Laser beam or beams can be used to heat and form the material bridge 336 to secure the ring member 304 to the outer tubular body 308. Directing laser welding energy toward or around the ring member 304 can result in damage to the sensor body 300 or optical fiber 220. Therefore the material bridge 336 is configured to protect or is formed in a manner that protects the sensor body 300 and the optical fiber 220 from damage in the coupling process, e.g., due to the laser welding.

A coupling zone 316 is provided on the pressure guidewire 116, in particular in the sensor housing section 180. The coupling zone 316 is configured in a manner that prevents the laser welding energy from potentially affecting the optical fiber 220. The coupling zone 316 can be limited to a portion of the cross-section of the sensor housing section 180 where the optical fiber 220 is not located, i.e. the coupling zone is offset from the central axis of the sensor housing section where the optical fiber 300 resides. The coupling zone 316 can be so limited in a method in which the ring member 304 is joined to the outer tubular body using a laser welding process. The laser can be directed in a direction that is toward an exterior surface of the ring member 304 but that is not in a direction toward the optical fiber 300.

A welding process can be defined that limits the location for application of energy within a boundary. The boundary can be defined as a portion of a cross-section of the sensor housing section 180 that does not intersect the optical fiber 220. The direction of the laser beam is offset from the central axis of the sensor housing where the optical fiber resides. The propagation of heat toward the optical fiber is therefore minimized.

The foregoing methods can be used to form the material bridge 336 (e.g., a weld line). The material bridge 336 is disposed between an inner surface of an outer tubular body 308 and an outer surface of the ring member 304. The outer tubular body 308 can be a span the tubular body that forms the middle section 148 and the outer surface of the sensor housing section 180. The material bridge 336 can span arc corresponding to an angle of at least 5 degrees of the outer surface of the ring member 304. The material bridge 336 can span an angle of at least 10 degrees of the outer surface of the ring member 304. The material bridge 336 can span an angle of at least 15 degrees of the outer surface of the ring member 304. The material bridge 336 can span an angle of at least 20 degrees of the outer surface of the ring member 304. The material bridge 336 can span an angle of at least 5 degrees of the inner surface of the outer tubular body 308. The material bridge 336 can span an angle of at least 10 degrees of the inner surface of the outer tubular body 308. The material bridge 336 can span an angle of at least 15 degrees of the inner surface of the outer tubular body 308. The material bridge 336 can span an angle of at least 20 degrees of the inner surface of the outer tubular body 308. The material bridge 336 can span an angle of between 5 degree and 90 degrees, between 10 degree and 70 degrees, and between 20 degree and 40 degrees.

The material bridge 336 can extend along an axial length of the ring member 304, e.g., along at least 30 percent of the length of the ring member 304. The material bridge 336 can extend at least 20 percent of the length of the ring member 304. The material bridge 336 can extend at least 10 percent of the length of the ring member 304.

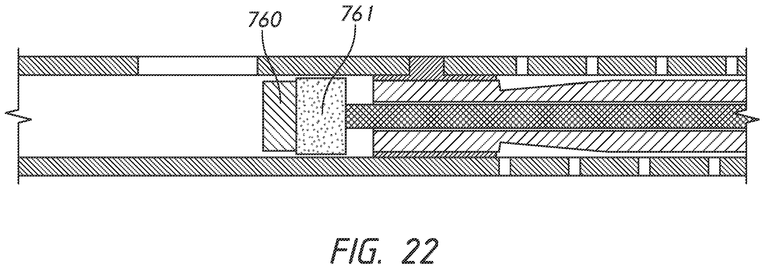

For a guidewire to be easily steered within a vasculature, it is desirable to have an advantageous, e.g., an optimal, flexibility profile, more specifically it is desirable to reduce or minimize a disruption of a continuous flexibility profile. Continuous flexibility profile can be achieved, among other specific flexibility profile parameters, by reducing or minimizing the length of stiff regions along the guidewire. The sensor housing primary function is to protect the sensor from external mechanical stress that may otherwise compromise the stability of measurements. Sensor housing stiffness is therefore a desirable feature. In order to reduce or minimize the impact of the sensor housing on the flexibility profile, sensor housing length should be reduced or minimized as much as possible. Shortening the overall length of the sensor assembly and ring member is therefore paramount in some embodiments. Sensor assembly illustrated in Fig. 22 allows further shortening of the sensor assembly and hence of the sensor housing. The MEMS device 760 can be a portion of the sensor that includes a diaphragm and the base supporting the diaphragm. The diaphragm can be made of silicon while the base can be made of glass, usually Pyrex^{®} or other glass compatible with anodic bonding to silicon. An advantageous sensor assembly would comprise the MEMS pressure portion 760 directly mounted on a ring member 761. The ring member is preferably made of metal that can be fused welded to the inside of the sensor housing or other tubular body. A preferred embodiment would be a MEMS pressure device bonded to a distal face of a ring member made of stainless steel 304 or other metal, the signal conductor or the optical fiber would be bonded inside and through the ring member to reach an optical contact with the MEMS proximal surface.

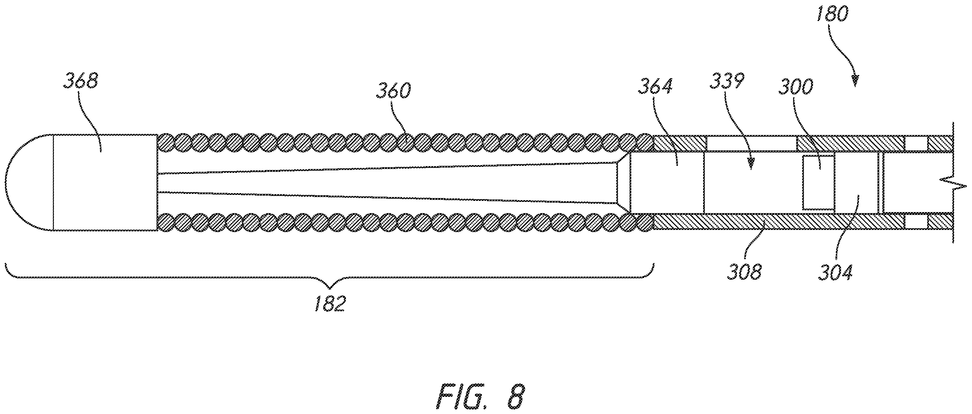

FIGS. 8-9 show the tip assembly 182 in greater detail. As discussed above the pressure guidewire 116 in inserted into the highly sensitive and delicate vasculature of a heart of a patient. Accordingly, the tip assembly 182 has to delicately engage the vascular tissue. Also, the tip assembly 182 has to be able to bend and flex in such interactions within kinking or fracturing. These requirements have resulted in very complex structures. The tip assembly 182 provides a streamlined design that at the same time protects the material properties of the components of the tip assembly 182.

The tip assembly 182 includes a core wire 364 disposed within a coil 360. The core wire 364 extends from a first (or proximal) end coupled with a distal end of the outer tubular body 308. FIG. 8 shows that the proximal end of the core wire 364 can be inserted into a distal opening of the outer tubular body 308. The proximal end of the core wire 364 can form a distal boundary of a sampling area 339 of the pressure guidewire 116. The sampling area 339 is an area in which blood can enter the pressure guidewire 116 and be sensed by the sensor body 300. The connection between the outer tubular body 308 and the proximal end of the core wire 364 can be done by any suitable technique, such as by laser welding.

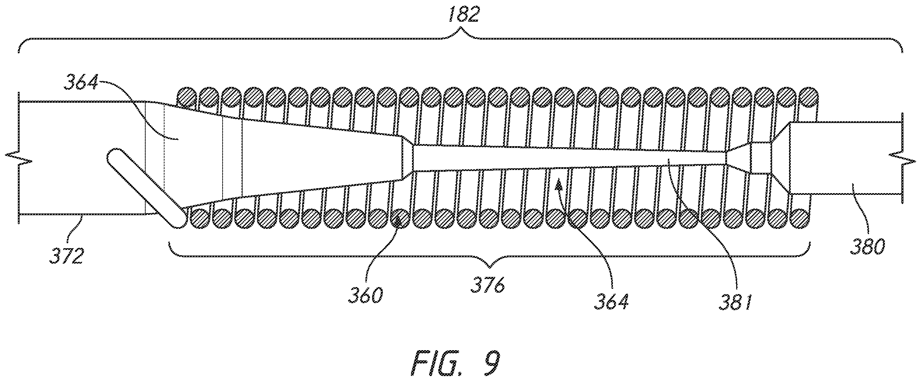

The core wire 364 can have a tapered profile from a proximal portion to a distal portion as shown in FIGS. 8 and 9. FIG. 8 shows a more simplified embodiment of the core wire 364. FIG. 9 shows that the core wire 364 can have a proximal portion 372 with a first outer diameter. The core wire 364 can have a profiled distal portion 376. The profiled distal portion 376 can include a first proximal taper and a second distal taper. The core wire 364 of FIG. 9 can have an aperture to receive an end portion of the coil 360. The proximal portion 372 can extend within the interior of the coil 360.

FIG. 9 shows that the profiled distal portion 376 causes the core wire 364 to reduce dramatically in diameter. A small diameter of the core wire 364 in the distal portion thereof can be less than one-half the diameter of the core wire 364 in the proximal portion 372. The small diameter of the core wire 364 in the distal portion thereof is less than one-fifth the diameter of the core wire 364 in the proximal portion 372. The small diameter of the core wire 364 in the distal portion thereof can be less than one-eighth the diameter of the core wire 364 in the proximal portion 372. The small diameter of the core wire 364 in the distal portion thereof is less than one-tenth the diameter of the core wire 364 in the proximal portion 372.

While the tapering of the profiled distal portion 376 provides desirable flexibility at the distal end of the tip assembly 182 the small diameter in the distal portion limits the options for the connection of the core wire 364 to an atraumatic tip member 368 of the tip assembly 182. This connection can be made by welding. Welding generates high heat that can degrade the performance of the core wire 364. The atraumatic tip member 368 presents a safe initial contact member for the pressure guidewire 116 as it advances through the vasculature. This can protect the vessel itself and also vulnerable plaque in the vessel, which the pressure guidewire 116 may have to engage and cross.

The core wire 364 is configured to enable the connection of the atraumatic tip member 368 thereto with a welding process while protecting the properties and performance of the core wire 364. Laser fusion welding can create the atraumatic tip member 368 from the core wire enlarged distal section 380 and the radiopaque coil 360. Due to the slender nature of the core wire 364 at the profiled distal portion 381 the heat generated by the welding process could potentially alter the material properties of the tip assembly 182. In particular, the core wire 364 is processed, e.g., cold worked to have high tensile and yield strengths to avoid fracture and unwanted plastic deformation. The heat of typical laser welding process would anneal the material to a point where these properties would be lost or compromised. The zone where the material properties are affected by the heating process is sometimes referred to herein as a heat affected zone (HAZ).

FIG. 9 shows that the core wire 364 can include a distal portion 380 that is configured to shield the slender portions of the profiled distal portion 376, e.g., the narrowest section(s) such that their desirable material properties are preserved. The distal portion 380 includes an enlarged member of a length configured to absorb heat in the process of creating the atraumatic tip member 368 from the melting of the distal portion 380 and the radiopaque coil 360. In one embodiment, the atraumatic tip member 368 has a recess configured to receive the distal portion 380. The distal portion 380 can be placed in the recess of the atraumatic tip member 368. After the distal portion 380 is advanced into the atraumatic tip member 368 energy can be applied to the distal portion 380 and the atraumatic tip member 368 to couple these components together. The size and length of the distal portion 380 prevents heat from propagating into the core wire reduced diameter section 381 by applying heat to the distal end of distal portion 380, hence preventing extreme heat from propagating and reaching the narrow portion 381 of the core wire 364. The distal portion 380 can be two times larger in diameter than the small diameter section of the profiled distal portion 376. The distal portion 380 can be three times larger in diameter than the small diameter section of the profiled distal portion 376. The distal portion 380 can be four times larger in diameter than the small diameter section of the profiled distal portion 376. The distal portion 380 can be five times larger in diameter than the small diameter section of the profiled distal portion 376. The distal portion 380 can be seven times larger in diameter than the small diameter section of the profiled distal portion 376. The narrowest portion of the profiled distal portion 376 can be maintained below a temperature of that corresponds to a melting temperature or below an annealing temperature, such as by way of non-limiting example 1000 degrees Celsius, for stainless 304 for example, while the distal portion 380 are melted with the radiopaque coil to create the atraumatic tip 368. The narrowest portion of the profiled distal portion 376 can be prevented from exceeding the annealing temperature of the material used to avoid any degradation of the ultimate tensile strength of the tip assembly 182

In another embodiment, the corewire 364 is formed or grinded with a profile that includes the distal portion 380. The distal portion 380 outside diameter is formed to fit within the coil 360. Clearance between coil and distal portion allows the formation of the atraumatic tip member 368 by melting and fusing together the distal end portion 380 with the portion of the coil that covers the distal portion 380. Any heat affected zone is kept away from the narrow portion 381 of the core wire 364 by fusing the distal end of the distal portion 380 to the coil. The distal portion is made of a length that results in a thermal gradient where the heat affected zone does not reach the proximal end of the distal end 380, and therefore the narrow portion 381 of the core wire 364.

The pressure guidewire 116 can also include a novel assembly for providing sealed flexibility in the middle section 148. The middle section 148 can be configured with a spiral, ribbon section, or coil configuration, as discussed above. The spiral section can be disposed around a middle portion of the hypotube 184 as discussed above. The spiral, ribbon, or coil can be enclosed, at least partially, in an outer sleeve 400. The outer sleeve 400 can be made of a suitable material. In one embodiment, the outer sleeve 400 is formed of PET. Other suitable materials can be used. FIG. 11 shows a portion of the middle section 148 with enhanced flexibility. The middle section 148 has a spiral cut portion, a ribbon or a coil as discussed above. FIG. 11 shows an outer portion of the middle section 148 and omits the hypotube 184 for clarity. The outer sleeve 400 includes an outer surface portion 404 and an expansion portion 408. The outer surface portion 404 includes a tubular member that is mounted to an outside surface of the spiral portion of the middle section 148. The expansion portion 408 can span between adjacent spiral sections of the spiral cut portion of the middle section 148. The expansion portion 408 can extend down into the gap between adjacent spirals of the ribbon spiral cut or coil portion. The expansion portion 408 can include a flexible span of material having a length greater than a gap between adjacent spiral sections of the spiral cut portion. The gap, in an undeflected state, can be any value between 0.01mm and 2.5mm, e.g., 0.01mm to 0.25mm, 0.015 to 0.05mm, 0.0254mm or any value at or between the end points of any of the foregoing ranges. The gap can vary from the foregoing nominal values in a deflected, e.g., curved or bent, state. These gap dimensions also apply to embodiments that include the spacing 502 and the spiral shaped gap 510 discussed above. The flexible span of the expansion portion 408 allows adjacent spirals of the spiral cut portion to move relative to each other such that the middle section 148 can flexibly and torque bend and thereafter contract to a straight configuration.

The outer sleeve 400 can be used to receive coating with specific characteristics along a portion of the middle section, such as hydrophilic coating or other coating. The outer sleeve 400 can be used to promote the adhesion of a coating on the outside surface of the middle section 148. The outer sleeve 400 can also be used to prevent matter, such as a coating, from reaching and getting into the interface between the spiral cut, ribbon or coil portion of the middle section 148 and the hypotube 184. The outer sleeve 400, while keeping the interface between the outside surface of the hypotube 184 and the inside surface of the middle section 148 free from coating, ensure the hypotube 184 can freely rotate relative the middle section 148, hence maintaining flexibility and torque transmission and

### Terminology

As used herein, the relative terms "proximal" and "distal" shall be defined from the perspective of the user of the system. Thus, proximal refers to the direction toward the user of the system and distal refers to the direction away from the user of the system.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments.

The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

The terms "approximately," "about," "generally," and "substantially" as used herein represent an amount close to the stated amount that still performs a desired function or achieves a desired result. For example, the terms "approximately," "about," "generally," and "substantially" may refer to an amount that is within less than 10% of the stated amount, as the context may dictate.

The ranges disclosed herein also encompass any and all overlap, sub-ranges, and combinations thereof. Language such as "up to," "at least," "greater than," "less than," "between" and the like includes the number recited. Numbers preceded by a term such as "about" or "approximately" include the recited numbers. For example, "about four" includes "four"

Any methods disclosed herein need not be performed in the order recited. The methods disclosed herein include certain actions taken by a practitioner; however, they can also include any third-party instruction of those actions, either expressly or by implication. For example, actions such as "distally moving a locking element" include "instructing distal movement of the locking element."

Although certain embodiments and examples have been described herein, it will be understood by those skilled in the art that many aspects of the humeral assemblies shown and described in the present disclosure may be differently combined and/or modified to form still further embodiments or acceptable examples. All such modifications and variations are intended to be included herein within the scope of this disclosure. A wide variety of designs and approaches are possible. No feature, structure, or step disclosed herein is essential or indispensable.

Some embodiments have been described in connection with the accompanying drawings. However, it should be understood that the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

For purposes of this disclosure, certain aspects, advantages, and novel features are described herein. It is to be understood that not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Moreover, while illustrative embodiments have been described herein, the scope of any and all embodiments having equivalent elements, modifications, omissions, combinations (e.g., of aspects across various embodiments), adaptations and/or alterations as would be appreciated by those in the art based on the present disclosure. The limitations in the claims are to be interpreted broadly based on the language employed in the claims and not limited to the examples described in the present specification or during the prosecution of the application, which examples are to be construed as non-exclusive. Further, the actions of the disclosed processes and methods may be modified in any manner, including by reordering actions and/or inserting additional actions and/or deleting actions. It is intended, therefore, that the specification and examples be considered as illustrative only, with a true scope and spirit being indicated by the claims and their full scope of equivalents.

The present application discloses subject matter in accordance with the following numbered clauses:
Clause 1. A pressure guidewire comprising:
   a shaft tube assembly comprising:
      a proximal section comprising a first tubular body comprising a proximal end, a distal end, a proximal outside surface and a proximal inside surface, the proximal inside surface enclosing a proximal portion of a central lumen, the proximal outside surface comprising an outer surface of the pressure guidewire;
      a middle section comprising a proximal end, a middle section outside surface, a middle section inside surface, the middle section inside surface disposed about a space within the pressure guidewire, the proximal end of the middle section being separate from and coupled to the distal end of the proximal section;
      a sensor housing section extending distally relative to the middle section;
   a hypotube comprising a proximal end portion and a distal end portion, the hypotube extending through the space about which the middle section inside surface is disposed, the proximal end portion of the hypotube coupled with the distal end of the proximal section and the distal end portion of the hypotube being coupled to the sensor housing; and
   a tip pressure sensor positioned in the sensor housing section.
Clause 2. The pressure guidewire of Clause 1, wherein the proximal inside surface comprises a first diameter and the middle section inside surface comprises a second diameter, the first diameter being less than the second diameter.
Clause 3. The pressure guidewire of Clause 1, wherein a first thickness is defined between the proximal inside surface and the proximal outside surface and a second thickness is defined between the middle section inside surface and the middle section outside surface, the first thickness being greater than the second thickness.
Clause 4. The pressure guidewire of Clause 1, wherein the tip pressure sensor comprises a signal conductor disposed through the middle section and through the proximal section, at least a portion of the signal conductor disposed inward of the proximal inside surface being centered on the a central longitudinal axis of the proximal section
Clause 5. The pressure guidewire of Clause 1, wherein the tip pressure sensor comprises a signal conductor disposed through the middle section and through the proximal section.
Clause 6. The pressure guidewire of Clause 1, wherein distal end of the of the proximal section comprises a first annular face disposed perpendicular to a longitudinal axis of the proximal section and the proximal end of the middle section comprises a second annular face disposed perpendicular to a longitudinal axis of the middle section, the first annular face and the second annular face contacting each other, a connection between the proximal section and the middle section comprising a weld.
Clause 7. The pressure guidewire of Clause 1, wherein the sensor housing section comprises a length of 2.5mm or less.
Clause 8. The pressure guidewire of Clause 1, further comprising a tip section having a length of less than 3.5 mm.
Clause 9. The pressure guidewire of Clause 1, wherein the middle section comprises a cut pattern configured to provide greater flexibility in the middle section than the proximal section.
Clause 10. The pressure guidewire of Clause 1, wherein the proximal end portion of the inner hypotube is joined to the proximal section proximal of a proximal end of the cut pattern, and wherein the distal end portion of the inner hypotube is joined to the sensor housing section distal of a distal end of the cut pattern.
Clause 11. The pressure guidewire of Clause 1, wherein the middle section comprises a spiral ribbon, a coil, and/or a laser cut pattern.
Clause 12. The pressure guidewire of Clause 11, further comprising a sleeve disposed over the spiral ribbon, the coil and/or the laser cut pattern, the sleeve configured to reduce or prevent ingress of matter into a space between the hypotube and the middle section or onto the hypotube.
Clause 13. The pressure guidewire of Clause 1, wherein the proximal end portion of the inner hypotube is joined to an inner surface in a lumen of the pressure guidewire, and wherein the distal end portion of the inner hypotube is joined to an inner surface in a lumen of the pressure guidewire proximal of the tip pressure sensor.
Clause 14. The pressure guidewire of Clause 1, wherein a distal end of the inner hypotube is positioned within the sensor housing section.
Clause 15. The pressure guidewire of Clause 1, wherein the inner hypotube comprises a tapered portion proximal to the distal end portion, the tapered portion being tapered in a distal direction.
Clause 16. The pressure guidewire of Clause 15, wherein the distal end portion of the hypotube is enlarged relative to the tapered portion.
Clause 17. The pressure guidewire of Clause 1, further comprising an optical fiber centered within the pressure guidewire in the proximal section and centered within the hypotube in the middle section.
Clause 18. The pressure guidewire of Clause 17, wherein the optical fiber is surrounded by a continuously concave surface in the proximal section.
Clause 19. A pressure guidewire comprising:
   a proximal end;
   a distal end;
   a proximal section extending from the proximal end of the pressure guidewire to a distal end of the proximal section;
   a sensor housing section disposed adjacent to the distal end of the pressure guidewire;
   an intermediate section disposed between the proximal section and the sensor housing section, the intermediate section having a proximal end separate from and coupled to the distal end of the proximal section;
   a tubular body comprising a proximal end portion and a distal end portion, the tubular body positioned within the intermediate section;
   a pressure sensor positioned in the sensor housing section and having a signal conductor disposed proximally of the sensor housing through the tubular body;
   wherein a wall thickness of the pressure guidewire being less in the intermediate section than in the proximal section to provide a stepped lumen profile.
Clause 20. The pressure guidewire of Clause 19, wherein the proximal section comprises an annular wall defining an inner diameter less than an outer diameter of the tubular body positioned within the intermediate section.
Clause 21. A guidewire assembly, comprising:
   a proximal section; and
   a distal section extending distally of the proximal section, the distal section comprising:
      an exterior metal body portion;
      a sensor assembly comprising a sensor body and a signal conductor coupled with the sensor body, the sensor assembly being disposed through the exterior body portion; and
      a metal ring member disposed between the exterior metal body portion and the signal conductor of the sensor assembly;
   wherein exterior metal body is joined to the metal ring member providing two metal layers around the sensor assembly.
Clause 22. The guidewire assembly of Clause 21, further comprising a weld line disposed about a portion of a periphery of the ring member.
Clause 23. The guidewire assembly of Clause 21, wherein the sensor body comprises an optical sensor.
Clause 24. The guidewire assembly of Clause 21, wherein the sensor body comprises a MEMS sensor.
Clause 25. The guidewire assembly of Clause 21, wherein the sensor body comprises an electrical sensor.
Clause 26. The guidewire assembly of Clause 21, wherein the ring member comprises a metal ring.
Clause 27. A method of forming a guidewire assembly, comprising:
   coupling a sensor body to a metal ring member;
   disposing the metal ring member within an exterior metal body; and
   joining a portion of an exterior surface of the metal ring member and a portion of an interior surface of the exterior metal body.
Clause 28. The method of Clause 27, wherein joining comprises laser welding an interface between the exterior surface of the ring member and the interior surface of the exterior body.
Clause 29. The method of Clause 27, wherein joining comprises directing a laser in a direction toward the exterior surface of the metal ring and/or the interior surface of the exterior metal body, the direction being offset from the central axis of the sensor body.
Clause 30. The method of Clause 27, wherein joining comprises soldering in a space between the exterior surface of the metal ring member and the interior surface of the exterior metal body.
Clause 31. A guidewire assembly, comprising:
   a proximal section having a proximal end and a distal end; and
   a distal portion comprising:
      a proximal end coupled with the distal end of the proximal section; and
      a detector disposed at or adjacent to a distal end of the distal portion;
   a junction comprising the distal end of the proximal section and the proximal end of the distal portion;
   wherein the junction comprises an enhanced ductility zone including a length of the distal portion including the proximal end thereof, a length of the proximal section portion including the distal end thereof, or a length of the distal portion including the proximal end thereof and a length of the proximal section including the distal end thereof.
Clause 32. The guidewire assembly of Clause 31, wherein the junction comprises a weld.
Clause 33. The guidewire assembly of Clause 31, wherein the junction comprises a heat treated portion.
Clause 34. The guidewire assembly of Clause 31, wherein the proximal section adjacent to the distal end thereof has a first thickness and the distal portion adjacent to a proximal end thereof has a second thickness different from the first thickness.
Clause 35. The guidewire assembly of Clause 31, wherein the first thickness is greater than the second thickness.
Clause 36. The guidewire assembly of Clause 31, wherein the detector comprises a pressure sensor.
Clause 37. The guidewire assembly of Clause 31, wherein the junction comprises a recess formed in a distal portion of the proximal section and a hypotube disposed in the recess, the hypotube extending from the recess into the proximal end of the distal portion.
Clause 38. The guidewire assembly of Clause 31, wherein the junction further comprises a hypotube extending distally of the proximal end of the distal portion and an adhesive bond with the hypotube, the adhesive bond extending into an aperture in a sidewall of the distal portion.
Clause 39. The guidewire assembly of Clause 31, wherein the junction further comprises a hypotube extending into the proximal end of the distal portion and into the proximal section, an adhesive bond coupled with the hypotube and with an aperture in the proximal section.
Clause 40. The guidewire assembly of Clause 31, wherein the junction further comprises a coupler disposed between the proximal section and the distal portion and a hypotube disposed within the coupler, the coupler joined by an adhesive bond to a hypotube.
Clause 41. A method of forming a pressure guidewire, comprising:
   providing a proximal body comprising a first tubular wall having a first wall thickness and a lumen of a first diameter;
   providing a distal body comprising a second tubular wall having a second wall thickness and a lumen of a second diameter, wherein the first diameter is smaller than the second diameter and the first wall thickness is greater than the second wall thickness;
   coupling a distal end of the proximal body to a proximal end of the distal body to provide a continuous assembly from proximal of the distal end of the proximal catheter body to distal of the proximal end of the distal catheter body;
   applying heat, after coupling, to the continuous assembly to enhance the ductility of at least a portion of the continuous assembly disposed at a location from proximal of the distal end of the proximal body to distal of the proximal end of the distal body.
Clause 42. The method of Clause 41, further comprising applying heat to the proximal body without directly applying heat to the distal body.
Clause 43. The method of Clause 41, wherein coupling comprises welding.
Clause 44. The method of Clause 41, wherein coupling comprises inserting a hypotube disposed in a proximal portion of the distal body into a recess formed in a distal portion of the proximal body.
Clause 45. The method of Clause 44, further comprising providing an adhesive bond with the hypotube and with an aperture in the distal body.
Clause 46. The method of Clause 44, further comprising providing an adhesive bond with the hypotube and with an aperture in the proximal body.
Clause 47. The method of Clause 44, further comprising providing an adhesive bond with the hypotube and a coupler disposed between the proximal body and the distal body.
Clause 48. A pressure guidewire, comprising:
   a shaft tube assembly; and
   a pressure sensor disposed in a distal portion of the shaft tube assembly, the pressure sensor coupled with a signal conduit to convey pressure signals to a processor;
   a tip assembly, comprising:
      a core wire having a proximal end coupled to a distal portion of the shaft tube assembly and an elongate tapered body having a lesser diameter toward a distal end thereof; and
      an atraumatic tip portion having a proximal end coupled with a distal end of the core wire and a rounded distal end, the proximal end configured to restrain heat gain at the distal end of the core wire to prevent a change in material properties in the distal end of the core wire.
Clause 49. The pressure guidewire of Clause 48, wherein the length of the atraumatic tip portion is at last three times the radius of the rounded distal end.
Clause 50. The pressure guidewire of Clause 48, wherein the radius of a proximal end of the atraumatic tip portion is at last two times the radius of the lesser diameter of the core wire.
Clause 51. A method of forming a pressure sensing guidewire, comprising:
   providing a shaft tube assembly having a distal portion with a pressure sensor disposed therein and a distal end;
   coupling a proximal end of a core wire with the distal end of the shaft tube assembly, the core wire having an elongate tapered body having a smaller size toward a distal end thereof than adjacent to a proximal end thereof, the core wire having a tip member disposed at the distal end of the elongate tapered body;
   positioning a coil over the core wire;
   coupling the coil to a proximal portion of the core wire;
   heating the tip member to melt a distal portion thereof to form an atraumatic tip portion having a convex shape;
   wherein the tip member has sufficient heat capacity to prevent material property changes in the core wire while allowing a distal portion to be formed having the convex shape following heating.
Clause 52. The method of Clause 51, wherein the atraumatic tip portion has a diameter at least two times the diameter of the distal end of the core wire.
Clause 53. The method of Clause 51, wherein the atraumatic tip portion has a length of at least two times a radius of curvature of the distal end of the atraumatic tip portion following the heating.
Clause 54. The method of Clause 51, wherein heating comprises directing laser energy to a distal portion of the tip member to melt the distal portion.

Nonetheless, for the avoidance of doubt, please note that the scope of the invention is to be defined by the appended claims.

## Claims

1. A pressure guidewire comprising:
a proximal end;
a distal end;
a proximal section extending from the proximal end of the pressure guidewire to a distal end of the proximal section;
a sensor housing section disposed adjacent to the distal end of the pressure guidewire;
an intermediate section disposed between the proximal section and the sensor housing section, the intermediate section having a proximal end separate from and coupled to the distal end of the proximal section;
a tubular body comprising a proximal end portion and a distal end portion, the tubular body positioned within the intermediate section;
a pressure sensor positioned in the sensor housing section and having a signal conductor disposed proximally of the sensor housing through the tubular body;
wherein a wall thickness of the pressure guidewire being less in the intermediate section than in the proximal section to provide a stepped lumen profile.

2. The pressure guidewire of Claim 1, wherein the proximal section comprises an annular wall defining an inner diameter less than an outer diameter of the tubular body positioned within the intermediate section.

3. The pressure guidewire of Claim 1 or Claim 2, further comprising a metal ring member disposed in the sensor housing section and coupled with the pressure sensor and the signal conductor, the metal ring member positioned adjacent to the pressure sensor.

4. The pressure guidewire of Claim 3, wherein a material bridge is provided between an outer surface of the metal ring member and an inside surface of the sensor housing section.

5. The pressure guidewire of any preceding Claim 1, wherein the signal conductor is an optical fiber.

6. The pressure guidewire of Claim 5, wherein the pressure sensor is an optical sensor.

7. The pressure guidewire of any preceding Claim, wherein the proximal section is configured to provide sufficient support without a core wire or reinforcement structure.

8. The pressure guidewire of any preceding Claim, further comprising a core wire having a proximal end coupled to the sensor housing section and an atraumatic tip portion having a proximal end coupled with a distal end of the core wire and a rounded distal end, the proximal end of the atraumatic tip portion configured to restrain heat gain at a distal end of the core wire to prevent a change in material properties in the distal end of the core wire.

9. The pressure guidewire of any preceding Claim, further comprising:
a junction comprising a distal end of the proximal section and the proximal end of the intermediate section;
wherein the junction comprises an enhanced ductility zone including a length of the intermediate section including the proximal end of the intermediate section, a length of the proximal section including the distal end of the proximal section, or a length of the intermediate section including the proximal end of the intermediate section and a length of the proximal section including the distal end of the proximal section.

10. The pressure guidewire of any preceding Claim, wherein the intermediate section comprises a spiral ribbon, a coil, and/or a laser cut pattern.

11. The pressure guidewire of Claim 10, further comprising a sleeve disposed over the spiral ribbon, the coil and/or the laser cut pattern, the sleeve configured to reduce or prevent ingress of matter into a space between the tubular body and the intermediate section or onto the tubular body.

12. A method of forming a guidewire assembly, comprising:
coupling a sensor body to a metal ring member;
disposing the metal ring member within an exterior metal body; and
joining a portion of an exterior surface of the metal ring member and a portion of an interior surface of the exterior metal body.

13. The method of Claim 12, wherein joining comprises laser welding an interface between the exterior surface of the ring member and the interior surface of the exterior body.

14. The method of Claim 12, wherein joining comprises directing a laser in a direction toward the exterior surface of the metal ring and/or the interior surface of the exterior metal body, the direction being offset from the central axis of the sensor body.

15. The method of Claim 12, wherein joining comprises soldering in a space between the exterior surface of the metal ring member and the interior surface of the exterior metal body.
